# EUROPEAN PATENT SPECIFICATION

(11) **EP 1 445 037 B1**
(45) Date of publication and mention of the grant of the patent: **08.10.2008**
(21) Application number: 04250526.3
(22) Date of filing: 30.01.2004
(51) Int. Cl.: B06B 1/06, H01L 41/22

(54) **System for repolarizing transducers in an ultrasonic probe**
System zur Repolarisierung von Wandlern in einer Ultraschallsonde
Système pour repolarizer des transducteurs dans une sonde ultrasonique

(30) Priority: 31.01.2003 JP 2003024554
(43) Date of publication of application: 11.08.2004
(73) Proprietor: KABUSHIKI KAISHA TOSHIBA, Minato-ku, Tokyo 105-8001 (JP); Toshiba Medical Systems Corporation, Otawara-shi, Tochigi (JP)
(72) Inventor: Shikata, Hiroyuki, Nasu-gun, Tochigi-ken (JP); Yuasa, Katsutoshi, Nasu-gun, Tochigi-ken (JP); Ogawa, Takashi, Nasu-gun, Tochigi-ken (JP); Makita, Yasuhisa, Nasu-gun, Tochigi-ken (JP)
(74) Representative: Maury, Richard Philip

(56) References cited:
- DE-A1- 10 155 894
- US-A- 5 810 009
- US-A- 5 938 615
- US-A- 6 087 762
- US-B1- 6 464 925
- PATENT ABSTRACTS OF JAPAN vol. 2000, no. 21, 3 August 2001 (2001-08-03) -& JP 2001 102651 A (TOSHIBA CORP), 13 April 2001 (2001-04-13)
- PATENT ABSTRACTS OF JAPAN vol. 1998, no. 12, 31 October 1998 (1998-10-31) -& JP 10 193601 A (MINOLTA CO LTD), 28 July 1998 (1998-07-28)

## Description

### BACKGROUND OF THE INVENTION

### Field of the invention

The present invention relates to a repolarization system which repolarizes transducers used in ultrasonic probe for generating ultrasound. The present invention further relates to ultrasound imaging apparatuses that use the repolarization system.

### Discussion of the Background

As known in the field of ultrasound imaging, an ultrasonic probe used as a part of ultrasound imaging apparatus includes a plurality of transducers. When the transducers are supplied with a high voltage, they generate ultrasound. The generated ultrasound is insonified towards an object or a patient. The insonified ultrasound returns from the object or the patient as an echo signal. The echo signal is converted into an electronic signal so as to obtain an ultrasound image. The obtained ultrasound image is displayed for observation. Such an ultrasound imaging apparatus is used, for example, as an ultrasound flaw detector (or a reflectoscope) for detecting flaws caused inside a welded part of metals. Further, such an ultrasound imaging apparatus is also used as an ultrasound diagnosis apparatus for examining the inside of a patient. The metal, the patient, or any other object to be insonified by the ultrasound imaging apparatus is hereinafter referred to as a specimen.

An ultrasonic probe used for an ultrasound imaging apparatus typically includes tens to hundreds of transducers arranged in an array. The number of transducers tends to increase as a higher resolution is required for images.

Each transducer is a provided with a pair of electrodes. When the pair of electrodes is supplied with a predetermined high voltage, the transducer generates an ultrasound. The polarization characteristic of the electrodes, however, usually deteriorates during the manufacture of the transducer and/or after a use for a predetermined time. As a resulc of the deterioration, the acoustic characteristic deteriorates in the ultrasonic probe. Accordingly, image quality deterioration is caused in a displayed ultrasound image.

In order to solve the above issue, various techniques have been introduced to restrain transducer depolarization (i.e., the deterioration of the polarization characteristic in a transducer). For example, Japanese Patent Application Publication No. PH7-99348 describes a piezoelectric monocrystal, an ultrasonic probe, and an array-type ultrasonic probe. Each of those can restrain the depolarization during the manufacture and the acoustic characteristic deterioration from long time use. According to the description, the depolarization is restrained by giving a specific condition to a monocrystal included in a transducer.

Another exemplary technique is described in Japanese Patent Application Publication No. PH10-93154. According to the description, it is possible to obtain a transducer having a high electromechanical coupling factor (or an acoustic characteristic) by restraining the depolarization of the transducer during the manufacture of the transducer. The restraint is accomplished by preparing the transducer with a Perovskite-type lead compound oxide monocrystal and giving a specific condition to the cutting surface shape of the transducer.

The techniques described above can be useful to restrain the depolarization of transducers and accordingly to postpone the depolarization. Once, however, the transducers are so depolarized that a preferred image quality cannot be kept any more, these techniques cannot cope with the problem of depolarization. Since there is no appropriate technique to solve this problem, the ultrasonic probe including the transducers must be replaced with a new one.

Meanwhile, it is generally known that a depolarized transducer can be repolarized by supplying a predetermined high voltage to its pair of electrodes. That is it ispossible to recover the acoustic characteristic of the transducer. A conventional ultrasonic probe is, however, not configured from the point of view of reusing its transducers by repolarization. Therefore, it is not possible to directly supply a predetermined high voltage to transducers because of the configuration of conventional ultrasonic probe. In order to supply the predetermined high voltage for repolarization, it is necessary to disassemble the ultrasonic probe, pick up the transducers, and then supply the predetermined high voltage to the transducers (or to the pair ot electrodes of each transducer). Such a configuration will be compared to an embodiment of the present invention in DESCRIPTION OF THE PREFERRED EMBODIMENTS" of the present invention.

Since the above repolarization method requires much work and even reassembly of the ultrasonic probe after the repolarization, it is not implemented in practice. In addition, a conventional ultrasound imaging apparatus has no means of informing when the depolarization is estimated to occur. Therefore, the operator has to determine a replacement time based on the image quality deterioration of displayed images.

JP 2001-102651A discloses a repolarization system including an ultrasonic probe comprising a plurality of electrodes provided in the ultrasonic probe, a plurality of transducers provided in the ultrasonic probe, each one of the transducers being connected to a pair of the electrodes, and a connector in the form of a flexible printed circuit board configured to connect the electrodes to a voltage supply for repolarization treatment.

US 6464925 discloses a system for a polarization treatment of a plurality of piezoelectric transducers comprising a voltage generator for polarization, a switch configured to control a supply of the polarization voltage to the electrodes, an interface configured to provide an instruction, and a controller configured to control the voltage generator to generate the predetermined voltage and to control the switch to supply the predetermined voltage to the electrodes in accordance with the instruction. A similar system for repolarizing a plurality of transducers comprising a voltage generator, a switch, a controller, and an interface is described in JP-10-193601A.

The invention provides a repolarization system and an ultrasound imaging apparatus as defined in Claim 1 and 16.

### BRIEF DESCRIPTION OF THE DRAWINGS

A more complete appreciation of embodiments of the present invention and many of its attendant advantages will be readily obtained by reference to the following detailed description considered in connection with the accompanying drawings, in which:
FIG. 1 is an illustration showing an exemplary configuration of an ultrasonic probe according to embodiments of the present invention;
FIGS. 2A and 2B are illustrations showing a first exemplary configuration for explaining a land connection according to embodiments of the present invention;
FIG 3 is an illustration showing a second exemplary configuration for explaining the land connection according to embodiments of the present invention;
FIG. 4 is an illustration showing an exemplary configuration for explaining a conventional land connection according to a prior art of the present invention;
FIG. 5 is a block diagram showing a first exemplary configuration of a repolarization system according to a first embodiment of the present invention;
FIG. 6 is a block diagram showing a second exemplary configuration of the repolarization system according to a second embodiment of the present invention;
FIG. 7 is a block diagram showing a third exemplary configuration of the repolarization system according to a third embodiment of the present invention;
FIG. 8 is a block diagram showing a fourth exemplary configuration of the repolarization system according to a fourth embodiment of the present invention;
FIG. 9 is a block diagram showing a fifth exemplary configuration of the repolarization system according to a fifth embodiment of the present invention;
FIG. 10 is a block diagram showing a sixth exemplary configuration of the repolarization system according to a sixth embodiment of the present invention;
FIG. 11 is a block diagram showing a first exemplary configuration of an ultrasound imaging apparatus according to a seventh embodiment of the present invention;
FIG. 12 is a flowchart showing an example of operations of an ultrasound imaging apparatus according to an eighth embodiment of the present invention; and
FIG. 13 is a block diagram showing a second exemplary configuration of the ultrasound imaging apparatus according to a ninth embodiment of the present invention.

### DESCRIPTION OF THE PREFERRED EMBODIMENTS

Embodiments of the present invention will be described with reference to the accompanying drawings.

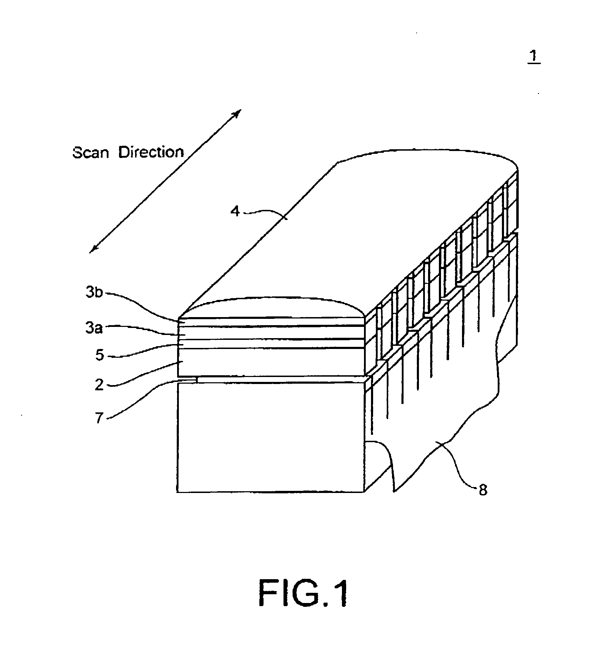

FIG. 1 is an illustration showing an exemplary configuration of an ultrasonic probe according to embodiments of the present invention. The ultrasonic probe can be used for an ultrasound imaging apparatus, such as, for example, an ultrasound flaw detector (or a reflectoscope) for detecting flaws caused inside a welded part of metals or an ultrasound diagnosis apparatus for the purpose of medical diagnoses.

As shown in FIG. 1, an ultrasonic probe 1 includes a plurality of transducers 2, acoustic matching layers 3a and 3b, an acoustic lens 4, electrodes 5 and 7, and a flexible printed wiring board 8. The transducers 2 are arranged in an array form and reversibly convert ultrasound signals to electronic signals, and vice versa. The transducers 2 are aligned along a scan direction of ultrasound generated from the transducers 2. The acoustic matching layers 3a and 3b are formed on an ultrasound reception surface side of the ultrasonic probe 1. Although the acoustic matching layers 3a and 3b are provided as a bilayer configuration in FIG. 1, a single layer or more than two layers may be applicable as an acoustic matching layer configuration. The acoustic matching layers 3a and 3b are provided over the transducers 2 along the scan direction. The acoustic matching layers 3a and 3b are covered by the acoustic lens 4.

The electrodes 5 are provided to the transducers 2, respectively and are individual electrodes for supplying or impressing (hereinafter referred to as supplying) a predetermined high voltage to the transducers 2, respectively. Further, the electrodes 7 are also provided to the transducers 2, respectively and are individual electrodes for taking out electronic signals from the transducers 2, respectively. Each transducer 2 is provided with one of the electrodes 5 and one of the electrodes 7, one of the electrodes 5 and one of the electrodes 7 can be in pairs. Alternatively, adjacent two or more of the transducers 2 may be provided with one of the electrodes 5 and one of the electrodes 7. One of the electrodes 5 and one of the electrodes 7 can be in pairs. In this alternative case, the adjacent two or more transducers 2, commonly provided with a pair of one of the electrodes 5 and one of the electrodes 7, operate as if they constitute one transducer.

Lead wires from the electrodes 7 are connected to the flexible printed wiring board 8 by soldering or conductive paste, for example. Lead wires from the electrodes 5 are also connected to the flexible printed wiring board 8 by soldering or conductive paste, for example. Alternatively, the electrodes 5 may be connected to an earth board while the lead wires from the electrodes 7 are connected to the flexible printed wiring board 8. The earth board may be connected to the flexible printed wiring board 8.

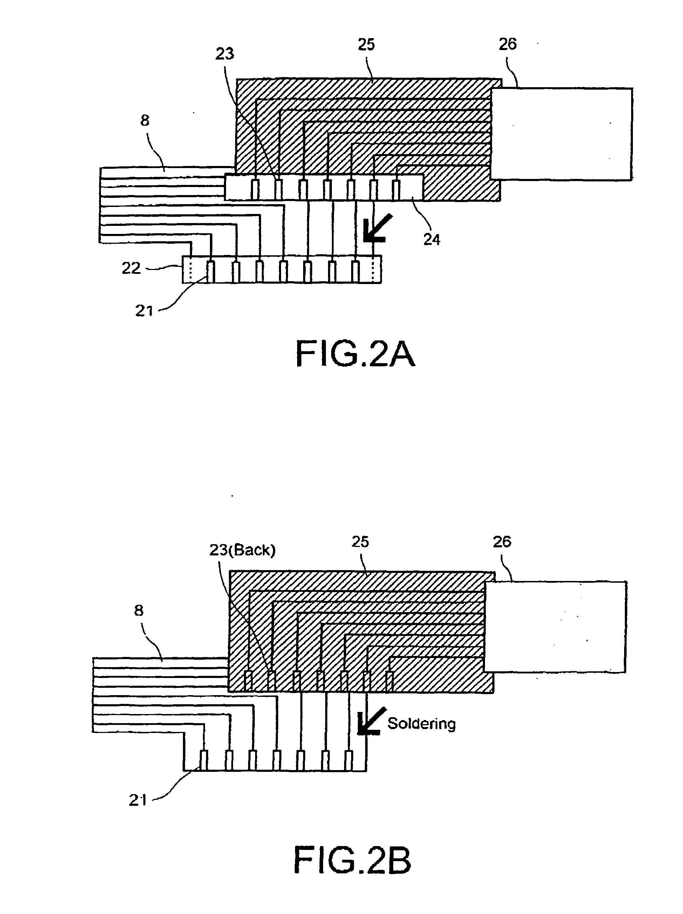

FIGS. 2A and 2B are illustrations showing a first exemplary configuration for explaining a land connection according to embodiments of the present invention. The land connection is implemented so as to connect the electrodes 5 and 7 (or transducers 2) and a cable of the ultrasonic probe 1.

As shown in FIG. 2A, the flexible printed wiring board 8 connected to the electrodes 5 and 7 has a connector 22. The connector 22 includes a plurality of lands 21. The lands 21 are connected to the electrodes 5 and 7 through voltage supply lines provided in the flexible printed wiring board 8. The lands 21 include signal lands and ground lands. Each signal land is connected to each of the electrodes 7. Each ground land is connected to one or more of the electrodes 5. In other words, one ground land can be used in common with a plurality of the electrodes 5.

In the connector 22, the lands 21 are provided at a predetermined interval. The predetermined interval is determined to be long enough to keep insulation between two adjacent lands 21 (i.e., between one of the lands 21 and a next of the one land 21). This is because a predetermined high voltage is supplied to the lands 21 so that the acoustic characteristic of the transducers 2 is recovered. The predetermined high voltage may be six to seven times larger than a voltage to be supplied to the transducers 2 for ultrasound generation. Therefore, if the creepage distance for insulation is not kept as a land interval, a dielectric breakdown is caused in the land interval. Such a dielectric breakdown leads to a breakage of the ultrasonic probe 1.

The electrodes 5 and 7 are supplied with the predetermined high voltage through the flexible printed wiring board 8, the lands 21 (the connector 22), lands 23 (a connector 24), a printed wiring board 25, and a cable 26. The connector 22 is connected to the connector 24. When the connectors 22 and 24 are connected to each other the lands 21 are connected to the lands 23. The lands 23 are prepared in a similar manner to the lands 21 so that each land 21 is connected to corresponding one of the lands 23. Each land 23 is also connected to one of voltage supply lines provided in the printed wiring board 25. The printed wiring board 25 is connected to the cable 26 which is also connected to an ultrasonic probe repolarization apparatus to be described later. The connection between the connector 22 and the connector 24 may be accomplished by soldering as shown in FIG. 2B so as to ensure the insulation between two adjacent lands 21 and also between corresponding two adjacent lands 23.

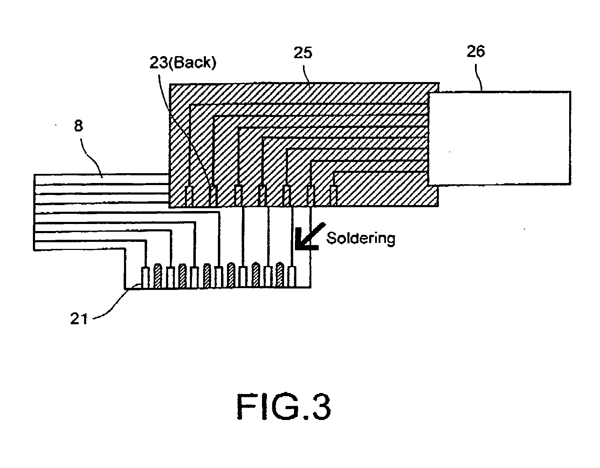

To avoid dielectric breakdown, an insulator may be provided between two adjacent lands 21 as shown in FIG. 3, instead of keeping the predetermined interval. Any other possible way can be used to avoid dielectric breakdown between two adjacent lands 21.

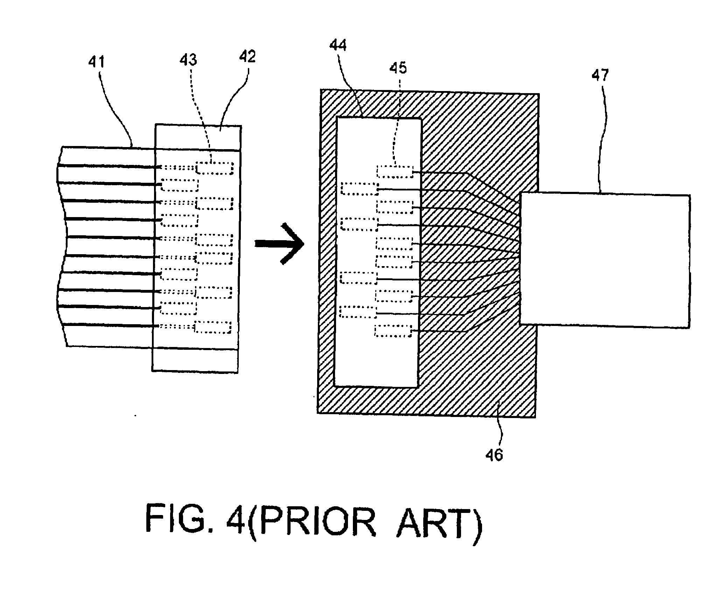

Compared to the above described land connection according to embodiments of the present invention, a conventional land connection will be described briefly with reference to FIG. 4. FIG. 4 is an illustration showing an exemplary configuration for explaining the conventional land connection according to prior art. Similar to FIG. 2A, a flexible printed wiring board 41 is connected to a connector 42 including a plurality of lands 43. Further, the connector 42 is connected to a connector 44 including a plurality of lands 45. The connector 44 provided in a printed wiring board 46 is also connected to a cable 47. The lands 43 provided in the connector 42 are connected to lands 45 provided in the connector 44. A typical feature of the lands 43 and 45 is a close-packed placement. In the connector 42, for example, two adjacent lands 43 are very close to each other. Consequently, if the predetermined high voltage is supplied to the lands 43, a dielectric breakdown is caused between the lands 43.

### (First Embodiment)

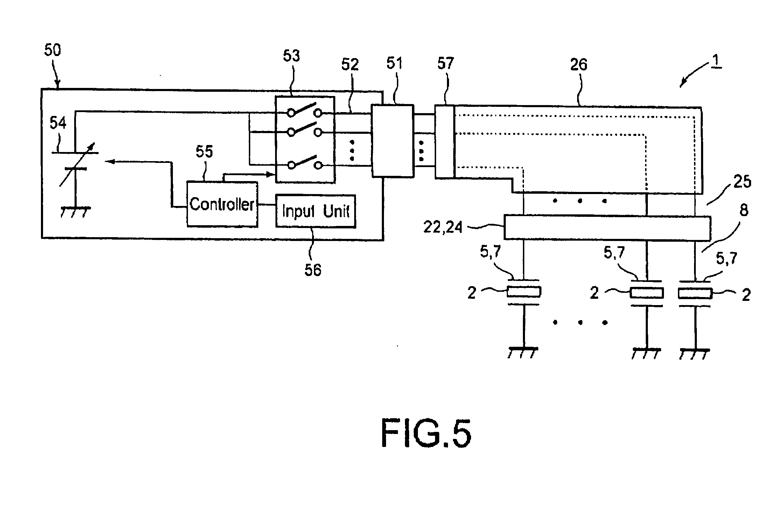

FIG. 5 is a block diagram showing a first exemplary configuration of a repolarization system according to a first embodiment of the present invention. As shown in a FIG. 5, the repolarization system includes an ultrasonic probe repolarization apparatus 50 and the ultrasonic probe 1. The ultrasonic probe repolarization apparatus 50 includes a connector 51, connection lines 52, a switch unit 53, a high voltage power source 54, a controller 55, and an input unit 56. The connector 51 is detachably connected to a connector 57 of the ultrasonic probe 1. The connector 57 is connected to the cable 26. Other configurations of the ultrasonic probe 1 are similar to chose described before. Therefore, an explanation of the ultrasonic probe 1 is omitted herein.

The switch unit 53 includes a plurality of switching elements. The switching elements are switched on/off by the controller 55. At least a portion of the switching elements corresponds to the lands 21 (23). Any of the switching elements corresponding to lands 21 (23) corresponds only one of the lands 21 (23) and no two switching elements correspond to the same land 21 (23). In other words, all the switching elements may not always be used since the number of the lands 21 (23) depends on the type of the ultrasonic probe 1. Each corresponding switching element is selected to switch on to supply a predetermined high voltage to a corresponding land 21 (23). The switch unit 53 is connected to the connector 51 through the connection lines 52. The predetermined high voltage is generated by the high voltage power source 54. The controller 55 controls the switch unit 53 to switch on for the switch on period of the switching elements. In addition the controller 55 also controls the high voltage power source 54 so that the predetermined high voltage is generated. The predetermined high voltage varies depending on the type of the ultrasonic probe 1. The input unit 56 is used to input the predetermined voltage value and/or the switch on period through an interface of the input unit 56. The controller 55 may further control the switch unit 53 and designate which switching elements to switch on (or which lands to be supplied with the predetermined high voltage). The controller 55 implements the above-described controls in accordance with the information input from the input unit 56.

The repolarization system configured as above will operate as follows.

An operator of the repolarization system inputs information of, for example, the predetermined high voltage and the switch-on period, from the input unit 56. The controller 55 receives the information and controls the high voltage power source 54 to generate the predetermined high voltage. The controller 55 also controls the switch unit 53 to switch on the switching elements for the input switch-on period.

The input unit 56 may alternatively include various buttons corresponding to various predetermined high voltages. Similarly, the input unit 56 may also include various buttons corresponding to various switch on periods which may be appropriate for the repolarization. In such cases, the operator only needs to press one of the buttons appropriate for each case. The buttons may alternatively be provided as icons in a display window if provided in the ultrasonic probe repolarization apparatus 50. The operator may click on one of the icons. Any of these inputs is construed as an instruction to the controller 55.

When the lands 21 (23) are supplied with the predetermined high voltage, the supplied voltage is applied to the electrodes 5 and 7. Since appropriate insulation is kept between one and the next of the lands 21 (23), the supplied high voltage is properly applied to the electrodes 5 and 7 for the switch-on period. Therefore, the transducers 2 are repolarized, which results in a recovery of the acoustic characteristic in the transducers 2. In response to the repolarization, the ultrasonic probe 1 can become reusable as an ultrasonic probe.

The predetermined high voltage may be determined according to the thickness of the transducers 2. The thickness of 1 millimeter may require a voltage 1 kilovolt. For example, 0.5 millimeter-thick transducers may need to be supplied with a 0.5-kilovolt voltage. In addition, a voltage supply period (i.e., the switch-on period) may be approximately 30 to 60 seconds whatever the thickness is, for example.

### (Second Embodiment)

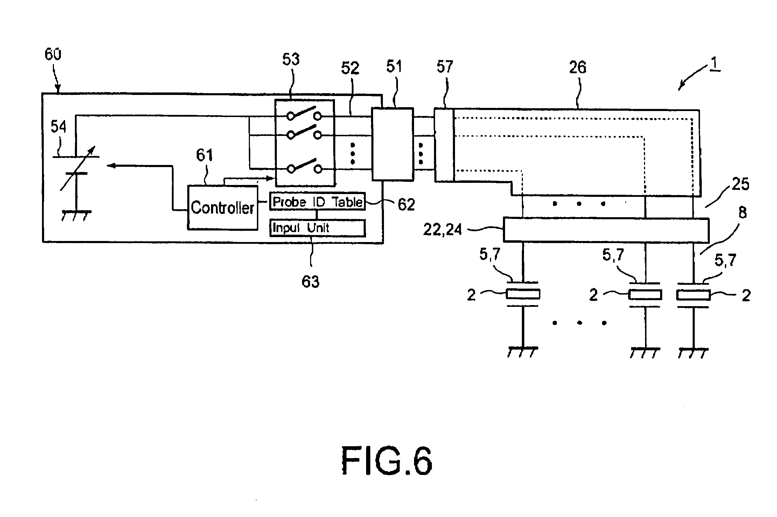

FIG. 6 is a block diagram showing a second exemplary configuration of the repolarization system according to a second embodiment of the present invention. In FIG. 6, components given the same reference numbers as chose shown in FIGS. 1, 2A, and 5 will be operative in similar manners. Therefore, detailed explanations of such components are omitted herein.

In the repolarization system according to the second embodiment, an ultrasonic probe repolarization apparatus 60 includes a controller 61 instead of the controller 55, an ultrasonic probe identification table memory 62, and an input unit 63 instead of the input unit 56. The controller 61 controls the switch unit 53 and designates which switching elements to switch on (or which lands to be supplied with the predetermined high voltage). The controller 61 further controls the switch unit 53 to switch on for the switch-on period of the switching elements. In addition, the controller 61 also controls the high voltage power source 54 so that the predetermined high voltage is generated. The predetermined high voltage varies depending on the type of the ultrasonic probe 1. The input unit 63 is used to input information identifying the type of the ultrasonic probe 1 through an interface of the input unit 63.

The ultrasonic probe identification table memory 62 stores a first table showing the relationship between ultrasonic probe type identification information and supply voltage information. The supply voltage information shows various voltages high enough co polarize the electrodes 5 and 7. Which of the voltages to supply depends on the type of the ultrasonic probe 1. Further, the ultrasonic probe identification table memory 62 also stores a second table showing the relationship between ultrasonic probe type identification information and switching condition information. The switching condition information may include switch-on periods of the switch unit 53 and information designating which switching elements to switch on. The switch-on period is determined to be long enough to polarize the electrodes 5 and 7. Which of the switch-on periods to apply depends on the type of the ultrasonic probe 1. It is necessary to determine the number of switching elements to switch on and/or to determine which switching elements to switch on since the number and relative positions of the lands 21 (23) are different among the different types of the ultrasonic probe 1. The first table and the second table may be combined to be used as one table. The ultrasonic probe repolarization apparatus 60 may alternatively include only one of the first and second tables. In this case, the input according to the first embodiment may be combined in the input operation. Still further, the ultrasonic probe identification table memory 62 may be provided in the controller 61 as a part of features of the controller 61. In this case the controller 61 refers to the ultrasonic probe identification table memory 62 for the controls.

The controller 61 implements the above-described controls in accordance with information supplied from the ultrasonic probe identification table memory 62 based on the information input by the input unit 63.

The repolarization system configured as above will operate as follows.

An operator of the repolarization system inputs information identifying the type of the ultrasonic probe 1 from the input unit 63. The ultrasonic probe identification table memory 62 receives the input information and compares the received information to the ultrasonic probe type identification information in the first table. As a result of the comparison, the supply voltage information corresponding to the ultrasonic probe type identification information identical to the received information is obtained and supplied to the controller 61. Similarly, the ultrasonic probe identification table memory 62 compares the received information to the ultrasonic probe type identification information in the second table. As a result of the comparison, the switching condition information corresponding to the ultrasonic probe type identification information identical to the received information is obtained and supplied to the controller 61. The controller 61 receives the supply voltage information and the switching condition information. The controller 61 controls the high voltage power source 54 to generate the predetermined high voltage based on the supply voltage information. Also based on the switching condition information, the controller 61 controls the switch unit 53 to switch on switching elements designated in the switching condition information for the switch-on period designated in the switching condition information. In case that the switch on period is fixed, the switch-on period is not required in the switching condition information.

The input unit 63 may alternatively include various buttons corresponding to various predetermined ultrasonic probe types. In such a case, the operator only needs to press the button corresponding to the appropriate type of the ultrasonic probe 1. The buttons may alternatively be provided as icons in a display window if provided in the ultrasonic probe repolarization apparatus 60. The operator may click one of the icons. Any of these inputs or information to be supplied from the ultrasonic probe identification table memory 62 is construed as an instruction to the controller 61.

Operations of the repolarization in the ultrasonic probe 1 are similar to those described in the first embodiment.

### (Third Embodiment)

When it is possible to categorize various types of the ultrasonic probe 1 into fewer number of groups in accordance with a supply voltage necessary for the repolarization, such categorized voltage types may be input to the controller of the ultrasonic probe repolarization apparatus to control the repolarization. This categorization is based on the fact that some types of ultrasonic probes can be repolarized with a similar voltage. Application of the controls according to such supply voltages may simplify operations by the operator and controls of the ultrasonic probe repolarization apparatus. One type of supply voltages corresponds to one predetermined voltage to be supplied for the repolarization.

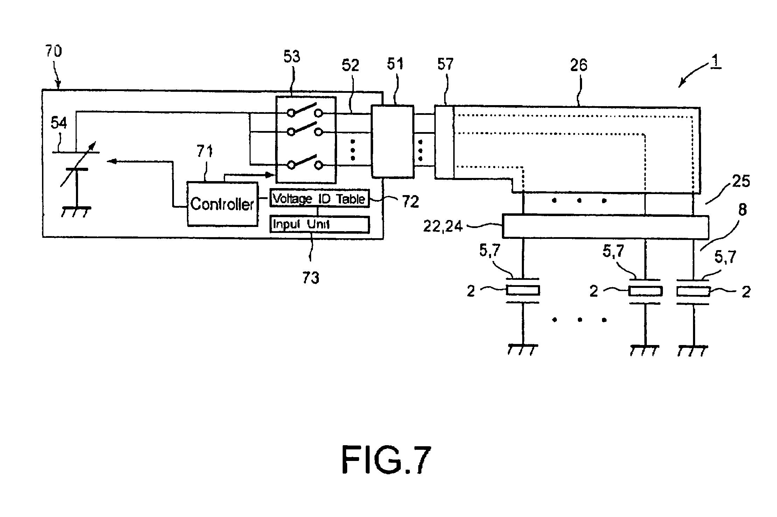

FIG. 7 is a block diagram showing a third exemplary configuration of the repolarization system according to a third embodiment of the present invention. In FIG. 7, components given the same reference numbers as those shown in FIGS. 1, 2A, and 5 will be operative in similar manners. Therefore, detailed explanations of such components are omitted herein.

In the repolarization system according to the third embodiment, an ultrasonic probe repolarization apparatus 70 includes a controller 71 instead of the controller 61, a supply voltage identification table memory 72, and an input unit 73 instead of the input unit 63. The controller 71 controls the switch unit 53 to switch on for a switch-on period of the switching elements. In addition, the controller 71 also controls the high voltage power source 54 so that the predetermined high voltage is generated. The predetermined high voltage varies depending on the type of the ultrasonic probe 1. The controller 71 may further control the switch unit 53 and designate which switching elements to switch on (or which lands to be supplied with the predetermined high voltage). The input unit 73 is used to input information identifying the supply voltage type of the predetermined high voltage for the ultrasonic probe 1 through an interface of the input unit 73.

The supply voltage identification table memory 72 stores a third table showing the relationship between supply voltage type identification information and supply voltage information. The supply voltage information shows various voltages high enough to polarize the electrodes 5 and 7. Which of the voltages to supply depends on the type of the ultrasonic probe 1. Further, the supply voltage identification table memory 72 also stores a fourth table shoving the relationship between supply voltage type identification information and switching condition information. The switching condition information may include switch on periods of the switch unit 53 and information designating which switching elements to switch on. The switch-on period is determined to be long enough to polarize the electrodes 5 and 7. Which of the switch-on periods to apply depends on the type of the ultrasonic probe 1. It is necessary to determine the number of switching elements to switch on and/or to determine which switching elements to switch on since the number and relative positions of the lands 21 (23) are different among the different types of the ultrasonic probe 1. The third table and the fourth table may be combined to be used as one table. The ultrasonic probe repolarization apparatus 70 may alternatively include only one of the third and fourth tables. In this case, the input according to the first embodiment may be combined in the input operation. Still further, the supply voltage identification table memory 72 may be provided in the controller 71 as a part of the features of the controller 71. In this case, the controller 71 refers to the supply voltage identification table memory 72 for the controls.

The controller 71 implements the above-described controls in accordance with information supplied from the supply voltage identification table memory 72 based on the information input by the input unit 73.

The repolarization system configured as above will operate as follows.

An operator of the repolarization system inputs information identifying the supply voltage type of the predetermined high voltage for the ultrasonic probe 1 from the input unit 73. The supply voltage identification table memory 72 receives the input information and compares the received information to the supply voltage type identification information in the third table. As a result of the comparison, the supply voltage information corresponding to the supply voltage type identification information identical to the received information is obtained and supplied co the controller 71. Similarly, the supply voltage identification cable memory 72 compares the received information to the supply voltage type identification information in the fourth cable. As a result of the comparison, the switching condition information corresponding to the supply voltage type identification information identical to the received information is obtained and supplied to the controller 71. The controller 71 receives the supply voltage information and the switching condition information. The controller 71 controls the high voltage power source 54 to generate the predetermined high voltage based on the supply voltage information. Also based on the switching condition information, the controller 71 controls the switch unit 53 to switch on switching elements designated in the switching condition information for the switch-on period designated in the switching condition information. In case that the switch-on period is fixed, the switch-on period is not required in the switching condition information.

The input unit 73 may alternatively includes various buttons corresponding to various predetermined supply voltage types. In such a case, the operator only needs to press the appropriate button for the predetermined type of the supply voltage. The buttons may alternatively be provided as icons in a display window if provided in the ultrasonic probe repolarization apparatus 70. The operator may click on one of the icons. Any of these inputs or information to be supplied from the supply voltage identification table memory 72 is construed as an instruction to the controller 71.

Operations of the repolarization in the ultrasonic probe 1 are similar to chose described in the first embodiment.

### (Fourth Embodiment)

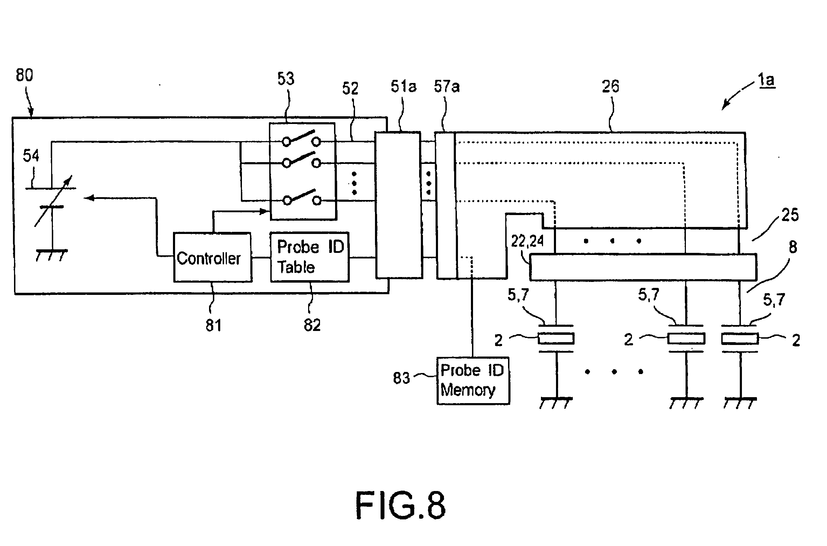

FIG. 8 is a block diagram showing a fourth exemplary configuration of the repolarization system according to a fourth embodiment of the present invention. In FIG. 8, components given the same reference numbers as those shown in FIGS. 1. 2A, and 5 will be operative in similar manners. Therefore, detailed explanations of such components are omitted herein.

In the repolarization system according to the fourth embodiment, an ultrasonic probe repolarization apparatus 80 includes a controller 81 instead of the controller 61, an ultrasonic probe identification table memory 82 instead of the ultrasonic probe identification cable memory 62, and a connector 51a instead of the connector 51. An ultrasonic probe la includes a connector 57a instead of the connector 57, and an ultrasonic probe identification memory 83.

The connector 57a is connected to the connector 51a. The connector 57a is also connected to the cable 26 and to the ultrasonic probe identification memory 83. The ultrasonic probe identification memory 83 stores information identifying the type of the ultrasonic probe 1a. The information can be transferred to the ultrasonic probe identification table memory 82 through the connector 57a and the connector 51a. The ultrasonic probe identification memory 83 may alternatively be provided in the connector 57a.

In the ultrasonic probe repolarization apparatus 80, the controller 81 controls the switch unit 53 and designates which switching elements to switch on (or which lands to be supplied with the predetermined high voltage). The controller 81 further controls the switch unit 53 to switch on for a switch-on period of the switching elements. In addition, the controller 81 also controls the high voltage power source 54 so that the predetermined high voltage is generated. The predetermined high voltage varies depending on the type of the ultrasonic probe 1a.

The ultrasonic probe identification table memory 82 stores a fifth table showing the relationship between ultrasonic probe type identification information and supply voltage information. The supply voltage information shows various voltages high enough to polarize the electrodes 5 and 7. Which of the voltages to supply depends on a type of the ultrasonic probe 1a. Further, the ultrasonic probe identification table memory 82 also stores a sixth table showing the relationship between ultrasonic probe type identification information and switching condition information. The switching condition information may include switch on periods of the switch unit 53 and information designating which switching elements to switch on. The switch-on period is determined to be long enough to polarize the electrodes 5 and 7. Which of the switch-on periods to apply depends on the type of the ultrasonic probe 1a. It is necessary to determine the number of switching elements to switch on and/or which switching elements to switch on since the number and relative positions of the lands 21 (23) are different among the different types of the ultrasonic probe 1a. The fifth table and the sixth table may be combined to be used as one cable. The ultrasonic probe repolarization apparatus 80 may alternatively include only one of the fifth and sixth tables. In this case, the input according to the first embodiment may be combined in the transferring operation as an input operation. Still further, the ultrasonic probe identification table memory 82 may be provided in the controller 81 as a part of the features of the controller 81. In this case, the controller 81 refers to the ultrasonic probe identification table memory 82 for the controls.

The controller 81 implements the above-described controls in accordance with the information supplied from the ultrasonic probe identification table memory 82 based on the information transferred from the ultrasonic probe identification memory 83 through the connector 57a and the connector 51a as interfaces.

According to the fourth embodiment of the present invention, the ultrasonic probe identification memory 83 may be provided in any part of the ultrasonic probe 1a.

The repolarization system configured as above will operate as follows.

An operator of the repolarization system connects the ultrasonic probe 1a to the ultrasonic probe repolarization apparatus 80 by putting the connector 57a into the connector 51a. In response to the connection or alternatively to a predetermined operation in the ultrasonic probe 1a and/or the ultrasonic probe repolarization apparatus 80, the information identifying the type of the ultrasonic probe 1a stored in the ultrasonic probe identification memory 83 is transferred to the ultrasonic probe identification table memory 82 through the connectors 57a and 51a. The ultrasonic probe identification table memory 82 receives the transferred information and compares the received information to the ultrasonic probe type identification information in the fifth cable. As a result of the comparison, the supply voltage information corresponding to the ultrasonic probe type identification information identical to the received information is obtained and supplied to the controller 81. Similarly, the ultrasonic probe identification table memory 82 compares the received information to the ultrasonic probe type identification information in the sixth cable. As a result of the comparison, the switching condition information corresponding to the ultrasonic probe type identification information identical to the received information is obtained and supplied to the controller 81. The controller 81 receives the supply voltage information and the switching condition information. The controller 81 controls the high voltage power source 54 to generate the predetermined high voltage based on the supply voltage information. Also based on the switching condition information, the controller 81 controls the switch unit 53 to switch on switching elements designated in the switching condition information for the switch-on period designated in the switching condition information. In case that the switch-on period is fixed, the switch-on period is not required in the switching condition information.

Operations of the repolarization in the ultrasonic probe 1a are similar to those described in the first embodiment.

### (Fifth Embodiment)

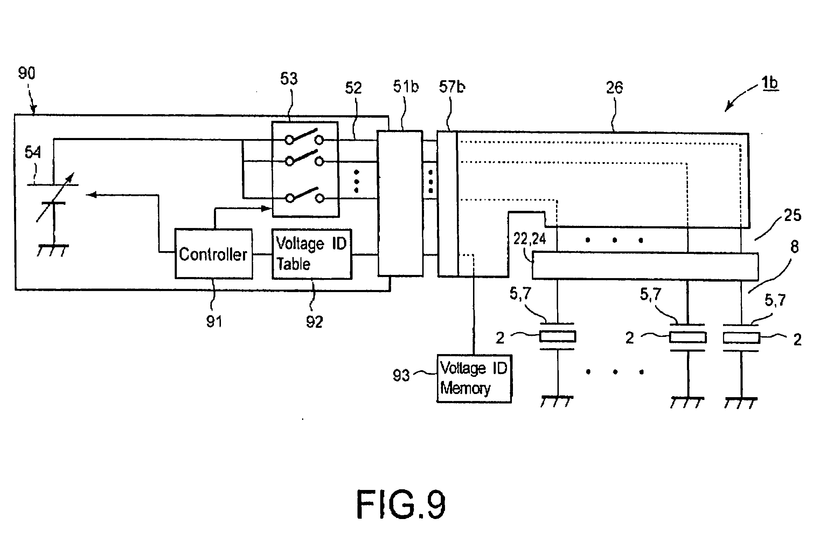

FIG. 9 is a block diagram showing a fifth exemplary configuration of the repolarization system according to a fifth embodiment of the present invention. In FIG. 9, components given the same reference numbers as those shown in FIGS. 1, 2A, and 5 will be operative in similar manners. Therefore, detailed explanations of such components are omitted herein.

In the repolarization system according to the fifth embodiment, an ultrasonic probe repolarization apparatus 90 includes a controller 91 instead of the controller 81, a supply voltage identification table memory 92 instead of the ultrasonic probe identification cable memory 82, and a connector 51b instead of the connector 51a. An ultrasonic probe 1b includes a connector 57b instead of the connector 57a, and a supply voltage identification memory 93 instead of the ultrasonic probe identification memory 83.

The connector 57b is connected to the connector 51b. The connector 57b is also connected to the cable 26 and to the supply voltage identification memory 93. The supply voltage identification memory 93 stores information identifying the supply voltage type of the ultrasonic probe 1b. The information can be transferred to the supply voltage identification table memory 92 through the connector 57b and the connector 51b. The supply voltage identification memory 93 may alternatively be provided in the connector 57b.

In the ultrasonic probe repolarization apparatus 90, the controller 91 controls the switch unit 53 and designates which switching elements to switch on (or which lands to be supplied with the predetermined high voltage). The controller 91 further controls the switch unit 53 to switch on for the switch-on period of the switching elements. In addition, the controller 91 also controls the high voltage power source 54 so that the predetermined high voltage is generated. The predetermined high voltage varies depending on the type of the ultrasonic probe 1b.

The supply voltage identification table memory 92 stores a seventh table showing the relationship between supply voltage type identification information and supply voltage information. The supply voltage information shows various a voltages high enough to polarize the electrodes 5 and 7. Which of the voltages to supply depends on the type of the ultrasonic probe 1b. Further, the supply voltage identification table memory 92 also stores an eighth table showing the relationship between supply voltage type identification information and switching condition information. The switching condition information may include switch-on periods of the switch unit 53 and information designating which switching elements to switch on. The switch-on period is determined to be long enough to polarize the electrodes 5 and 7. Which of the switch-on periods to apply depends on the type of the ultrasonic probe 1b. It is necessary to determine the number of switching elements to switch on and/or to determine which switching elements to switch on since the number and relative positions of the lands 21 (23) are different among the different types of the ultrasonic probe 1b. The seventh table and the eighth table may be combined to be used as one table. The ultrasonic probe repolarization apparatus 90 may alternatively include only one of the seventh and eighth tables. In this case, the input according to the first embodiment maybe combined in the transferring operation as an input operation. Still further, the supply voltage identification table memory 92 may be provided in the controller 91 as a part of the features of the controller 91. In this case, the controller 91 refers to the supply voltage identification table memory 92 for the controls.

The controller 91 implements the above-described controls in accordance with information supplied from the supply voltage identification table memory 92 based on the information transferred from the supply voltage identification memory 93 through the connector 57b and the connector 51b as interfaces.

According to the fifth embodiment of the present invention, the supply voltage identification memory 93 may be provided in any part of the ultrasonic probe 1b.

The repolarization system configured as above will operate as follows.

An operator of the repolarization system connects the ultrasonic probe 1b to the ultrasonic probe repolarization apparatus 90 by putting the connector 57b into the connector 51b. In response to the connection or alternatively to a predetermined operation in the ultrasonic probe 1b and/or the ultrasonic probe repolarization apparatus 90. the information identifying the supply voltage type of the ultrasonic probe 1b stored in the supply voltage identification memory 93 is transferred to the supply voltage identification table memory 92 through the connectors 57b and 51b. The supply voltage identification table memory 92 receives the transferred information and compares the received information to the supply voltage type identification information in the seventh table. As a result of the comparison, the supply voltage information corresponding to the supply voltage type identification information identical to the received information is obtained and supplied to the controller 91. Similarly, the supply voltage identification table memory 92 compares the received information to the supply voltage type identification information in the eighth table. As a result of the comparison, the switching condition information corresponding to the supply voltage type identification information identical to the received information is obtained and supplied to the controller 91. The controller 91 receives the supply voltage information and the switching condition information. The controller 91 controls the high voltage power source 54 to generate the predetermined high voltage based on the supply voltage information. Also based on the switching condition information, the controller 91 controls the switch unit 53 to switch on switching elements designated in the switching condition information for the switch-on period designated in the switching condition information. In case that the switch-on period is fixed, the switch-on period is not required in the switching condition information.

Operations of the repolarization in the ultrasonic probe 1b are similar to those described in the first embodiment.

### (Sixth Embodiment)

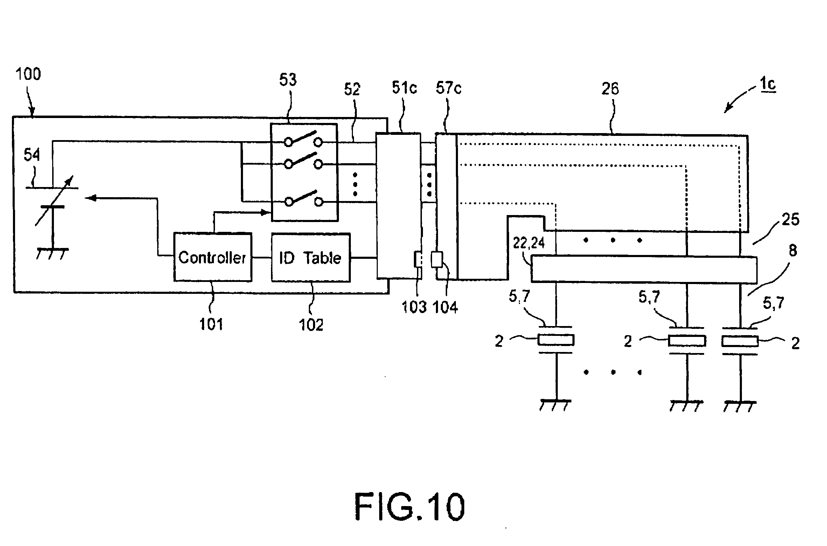

FIG. 10 is a block diagram showing a sixth exemplary configuration of the repolarization system according to a sixth embodiment of the present invention. In FIG. 10. components given the same reference numbers as those shown in FIGS. 1, 2A, and 5 will be operative in similar manners. Therefore, detailed explanations of such components are omitted herein.

In the repolarization system according to the sixth embodiment, an ultrasonic probe repolarization apparatus 100 includes a controller 101 instead of the controller 91, an identification table memory 102 instead of the supply voltage identification table memory 92, a first mechanism 103, and a connector 51c instead of the connector 51b. An ultrasonic probe 1c includes a connector 57c instead of the connector 57b and a second mechanism 104.

The connector 57c is connected to the connector 51c. The connector 57c is also connected to the cable 26. The second mechanism 104 is provided in the connector 57c. The second mechanism 104 is mechanically configured to have joint indicating predetermined information. The predetermined information is, for example, the ultrasonic probe type or the supply voltage type of the ultrasonic probe 1c. The second mechanism 104 is detachably attached to the first mechanism 103. The first mechanism 103 is provided in the connector 51c. The first mechanism 103 is configured to have joint operative to respond to and recognize the first mechanism 103 and supply the identification table memory 102 with the predetermined information in accordance with the recognized mechanism. That is, electronic signals representing the predetermined information are generated in response to the operation of the first mechanism 103.

In the ultrasonic probe repolarization apparatus 100, the controller 101 controls the switch unit 53 and designates which switching elements to switch on (or which lands to be supplied with the predetermined high voltage). The controller 101 further controls the switch unit 53 to switch on for the switch-on period of the switching elements. In addition, the controller 101 also controls the high voltage power source 54 so that the predetermined high voltage is generated. The predetermined high voltage varies depending on the type of the ultrasonic probe 1c.

The identification table memory 102 stores a ninth table showing the relationship between identification information and supply voltage information. The supply voltage information shows voltages high enough co polarize the electrodes 5 and 7. Which of the voltages to supply depends on the type of the ultrasonic probe 1c. Further, the identification table memory 102 also stores a tenth table showing the relationship between identification information and switching condition information. The switching condition information may include switch-on periods of the switch unit 53 and information of designating which switching elements to switch on. The switch-on period is determined to be long enough to polarize the electrodes 5 and 7. Which of the switch-on periods to apply depends on the type of the ultrasonic probe 1c. It is necessary to determine the number of switching elements to switch on and/or to determine which switching elements to switch on since the number and relative positions of the lands 21 (23) are different among the different types of the ultrasonic probe 1c The ninth table and the tenth table may be combined to be used as one table. The ultrasonic probe repolarization apparatus 100 may alternatively include only one of the ninth and tenth tables. In this case, the input according to the first embodiment may be combined in the attachment operation as an input operation. Still further, the identification cable memory 102 may be provided in the controller 101 as a part oE the features of the controller 101. In this case, the controller 101 refers to the identification table memory 102 for the controls.

The controller 101 implements the above-described controls in accordance with information supplied from the identification table memory 102 based on the predetermined information recognized by the first mechanism 103 in response to the second mechanism 104.

The repolarization system configured as above will operate as follows.

An operator of the repolarization system connects the ultrasonic probe 1c to the ultrasonic probe repolarization apparatus 100 by putting the connector 57c into the connector 51c. In response to the connection, the second mechanism 104 is attached to the first mechanism 103. Accordingly, the predetermined information based on the second mechanism 104 is recognized by the first mechanism 103. Electronic signals representing the recognized predetermined information are supplied to the identification cable memory 102. The identification table memory 102 receives the supplied information and compares the received information to the identification information in the ninth table. As a result of the comparison, the supply voltage information corresponding to the identification information identical to the received information is obtained and supplied to the controller 101. Similarly, the identification cable memory 102 compares the received information to the identification information in the tenth table. As a result of the comparison, the switching condition information corresponding to the identification information identical to the received information is obtained and supplied to the controller 101. The controller 101 receives the supply voltage information and the switching condition information. The controller 101 controls the high voltage power source 54 to generate the predetermined high voltage based on the supply voltage information. Also based on the switching condition information, the controller 101 controls the switch unit 53 to switch on switching elements designated in the switching condition information for the switch-on period designated in the switching condition information. In case that the switch-on period is fixed, the switch-on period is not required in the switching condition information.

Operations of the repolarization in the ultrasonic probe 1c are similar to those described in the first embodiment.

### (Seventh Embodiment)

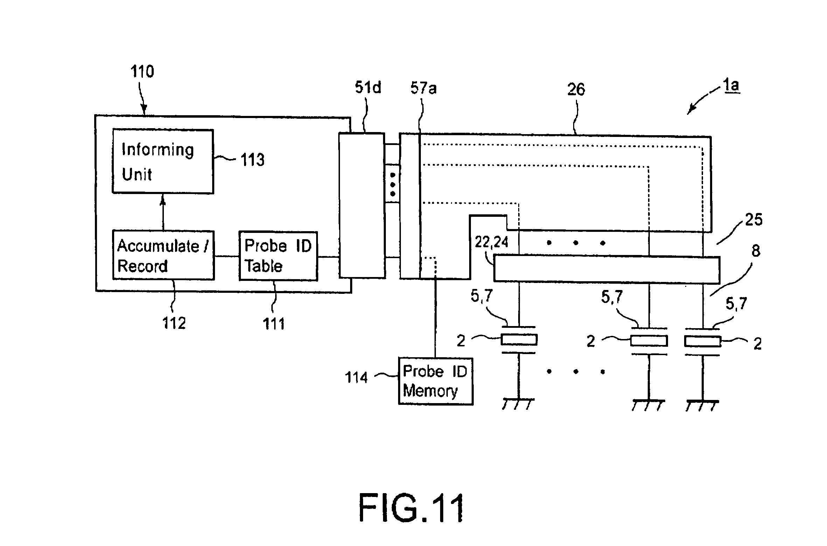

FIG. 11 is a block diagram showing a first exemplary configuration of an ultrasound imaging apparatus according to a seventh embodiment of the present invention. The ultrasound imaging apparatus includes a main body 110 and the ultrasonic probe 1a. Components of the ultrasonic probe 1a are similar co chose of the ultrasonic probe 1a shown in FIG. 8. Therefore explanations of such components will be omitted herein. The main body 110 includes an ultrasonic probe identification table memory 111, an accumulator 112, an informing unit 113, and a connector 51d. In addition, the main body 110 further includes a controller, a switch unit, a high voltage power source, and an input unit, which are not shown in PIG. 11. Needless to say, the main body 110 also includes components typically required for a conventional ultrasound imaging apparatus.

The ultrasonic probe 1a is detachably attached to the main body 110 through the connectors 57a and 51d. The ultrasonic probe identification memory 114 stores information identifying the ultrasonic probe 1a. Information identifying the ultrasonic probe 1a stored in the ultrasonic probe identification memory 114 can be transferred to the ultrasonic probe identification table memory 111 through the connector 57a and the connector 51d.

In the main body 110, the controller controls the switch unit and designates which switching elements to switch on (or which lands to be supplied with a predetermined high voltage). The controller further controls the switch unit to switch on for the switch-on period of the switching elements. In addition, the controller also controls the high voltage power source so that the predetermined high voltage is generated. The predetermined high voltage varies depending on the type of the ultrasonic probe 1a.

The ultrasonic probe identification table memory 111 stores an eleventh table showing the relationship between ultrasonic probe identification information and supply voltage information. The supply voltage information shows various voltages high enough to polarize the electrodes 5 and 7. Which of the voltages to supply depends on the type of the ultrasonic probe 1a. Further, the ultrasonic probe identification table memory 111 also stores a twelfth table showing the relationship between ultrasonic probe identification information and switching condition information. The switching condition information may include switch-on periods of the switch unit and information designating which switching elements to switch on. The switch-on period is determined to be long enough to polarize the electrodes 5 and 7. Which of the switch-on periods to apply depends on the type of the ultrasonic probe 1a. It is necessary to determine the number of switching elements to switch on and/or to determine which switching elements to switch on since the number and relative positions of the lands 21 (23) are different among the different types of the ultrasonic probe 1a. The eleventh table and the twelfth table may be combined to be used as one table. The main body 110 may alternatively include only one of the eleventh and twelfth tables. In this case, inputs in a manner similar to the first embodiment may be combined in the transferring operation as an input operation. Still further, the ultrasonic probe identification table memory 111 may be provided in the controller as a part of the features of the controller. In this case, the controller refers to the ultrasonic probe identification table memory 111 for the controls.

The accumulator 112 accumulates time during the use of the ultrasonic probe 1a. The use of the ultrasonic probe 1a may be defined, for example, as the ultrasonic probe 1a being supplied with a power to generate an ultrasound. Further, for example, the use of the ultrasonic probe 1a may indicate that the ultrasonic probe 1a generates an ultrasound. The accumulated time is stored in a recorder provided in the accumulator 112. The recorder may alternatively be provided independently or in the controller. The accumulation by the accumulator 112 is implemented every ultrasonic probe identification information. This is because the ultrasonic probe 1a may often be changed to others among a plurality of ultrasonic probes in accordance with a requirement in an ultrasound imaging.

The informing unit 113 gives a notice to an operator when the accumulated time has reached a predetermined time. The notice may be a suggestion that the ultrasonic probe 1a (i.e., the transducers 2) should be repolarized. The informing unit 113 may alternatively give a notice as a warning at a predetermined time before the time when the ultrasonic probe 1a should be repolarized. In this case, the notice may indicate when the ultrasonic probe 1a should be repolarized. Such an indication may be displayed as a message in a display provided for displaying ultrasound images as a typical component of the ultrasound imaging apparatus. The ultrasound imaging apparatus may alternatively warn it with a sound, such as, for example, beep sound or a voice message. Instead of when to repolarize, the remaining time until when the ultrasonic probe 1a should be repolarized may be calculated or estimated based on the accumulated time and be displayed in the display or be given as a sound warning.

Usually the predetermined time is determined based on the image quality of images displayed in the display. If the depolarization progresses to a certain extent, the image quality deteriorates beyond the permissible quality range. The predetermined time may be a time when such deterioration is caused and may depend on the type of the ultrasonic probe 1a or the transducers 2. Typically, the predetermined time may be about one year.

When the operator inputs an instruction through the input unit in response to the notice given by the informing unit 113, the controller controls the switch unit and the high voltage power source so that the repolarization is implemented as described before.

### (Eighth Embodiment)

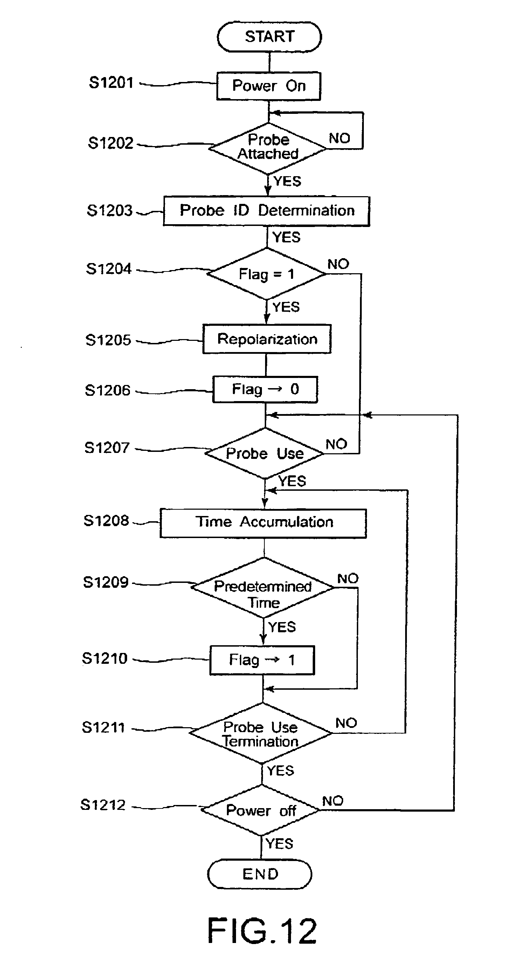

FIG. 12 is a flowchart showing an example of operations of an ultrasound imaging apparatus according to an eighth embodiment of the present invention. The ultrasound imaging apparatus according to the eighth embodiment will be described with reference to FIGS. 11 and 12. The ultrasound imaging apparatus is, however, not necessarily required to incorporate the informing unit 113 according to the eighth embodiment.

The ultrasound imaging apparatus automatically implements the repolarization under a predetermined condition as shown in FIG. 12. Operations shown in steps of FIG. 12 are implemented by the controller, the ultrasonic probe identification table memory 111, the accumulator 112, and so on. A flag is set to zero as an initial state when the ultrasonic probe 1a is used for the first time after purchase.

When the ultrasound imaging apparatus is switched on for operation (step S1201), it is determined whether the ultrasonic probe 1a is attached to the main body 110 or not (step S1202). In response to the determination of the attachment, ultrasonic probe identification information of the ultrasonic probe 1a is transferred from the ultrasonic probe 1a to the main body 110and recognized (step S1203).After the recognition, the flag stored in correspondence with the recognized ultrasonic probe identification information is determined whether it is one or zero (step S1204). If the flag is one, the main body 110 of the ultrasound imaging apparatus automatically implements the repolarization on the ultrasonic probe 1a (or the transducers 2) (step S1205). Then, the flag is set to zero (step S1206).

After step S1206 and when the flag is not one in step S1204, it is determined whether the ultrasonic probe 1a is being used or not (step S1207). In the determination of the use of the ultrasonic probe 1a, time accumulation is implemented (step S1208). The accumulated time is recorded in the recorder. When the accumulated time has reached a predetermined time (step S1209), the flag is changed co one (step S1210). The predetermined time may be set, taking into consideration, for example, average time estimated to be required for one day use of the ultrasonic probe 1a as a margin. The time accumulation continues during the use of the ultrasonic probe 1a. When the use of the ultrasonic probe 1a is terminated (step S1211), the time accumulation is terminated. Further, in response to switching off the power supply to the ultrasound imaging apparatus, the operations are terminated (step S1212). The accumulated and stored time is kept until the next use of the ultrasonic probe 1a. The flag for the ultrasonic probe 1a is also stored and kept until the next use of the ultrasonic probe 1a.

According to the operations described above, when the ultrasonic probe 1a is used and accumulated time has reached the predetermined time, the repolarization is automatically implemented at the beginning of the next use of the ultrasonic probe 1a. Therefore, the operator needs neither to pay attention to the time for the repolarization nor to perform a predetermined operation for the repolarization.

As a first alternative example,when it is determined that the accumulated time has reached a predetermined time in step S1209, the repolarization may be automatically implemented in response to the termination of the use of the ultrasonic probe 1a in step S1211.

Still further, it can be a second alternative example that an operator's confirmation is requested by displaying such a confirmation request message in the display or by a voice message in advance of implementing the repolarization in step S1205 or in the above first alternative example.

### (Ninth Embodiment)

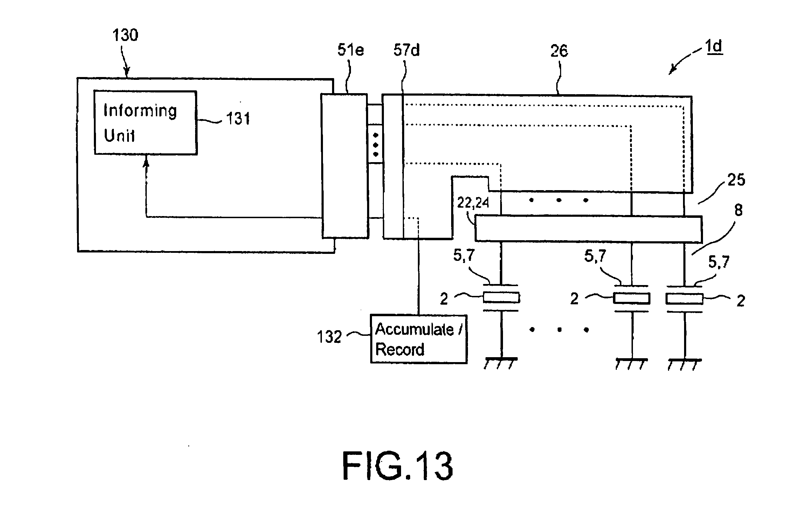

FIG. 13 is a block diagram showing a second exemplary configuration of the ultrasound imaging apparatus according to a ninth embodiment of the present invention. In FIG. 13, components given the same reference numbers as those shown in FIGS. 1 and 2A, and 5 will be operative in similar manners. Therefore, detailed explanations of such components are omitted herein.

The ultrasound imaging apparatus includes a main body 130 and an ultrasonic probe 1d. The main body 130 includes an informing unit 131 and a connector 51e. In addition, the main body 130 further includes a controller, a switch unit, a high voltage power source, an ultrasonic probe identification table memory, and an input unit, which are not shown in FIG. 13. Needless to say, the main body 130 also includes components typically required for a conventional ultrasound imaging apparatus. The ultrasonic probe 1d includes a connector 57d, and an accumulator 132, in addition to the components shown in FIGS. 1 and 2A.

The connector 57d is connected to the connector 51e. The connector 57d is also connected to the cable 26 and to the accumulator 132. Therefore, the ultrasonic probe 1d is detachably connected (attached) to the main body 130 through the connectors 57d and 51e. The accumulator 132 accumulates time during the use of the ultrasonic probe 1d. The accumulated time is stored in a recorder provided in the accumulator 132. The recorder may alternatively be provided independently in the ultrasonic probe 1d or in the main body 130. The accumulator 132 may alternatively be provided in the connector 57d.

The accumulated time is transferred to the controller provided in the main body 130. The controller determines whether the accumulated time has reached a predetermined time. Alternatively, when it is determined in the accumulator 132 that the accumulated time has reached the predetermined time, instruction signals may be sent to the informing unit 130 through the connector 57d and the connector 51e.

In the main body 130, the controller controls the switch unit and designates which switching elements to switch on (or which lands to be supplied with a predetermined high voltage). The controller further controls the switch unit to switch on for the switch-on period of the switching elements. In addition, the controller also controls the high voltage power source so that the predetermined high voltage is generated. The predetermined high voltage varies depending on the type of the ultrasonic probe 1d.

The ultrasonic probe identification table memory stores a table showing the relationship between ultrasonic probe identification information and supply voltage information. The supply voltage information shows various voltages high enough to polarize the electrodes 5 and 7. Which of the voltages to supply depends on the type of the ultrasonic probe 1d. Further, the ultrasonic probe identification table memory also stores another cable showing the relationship between ultrasonic probe identification information and switching condition information. The switching condition information may include switch-on periods of the switch unit and information designating which switching elements to switch on. The switch-on period is determined to be long enough to polarize the electrodes 5 and 7. Which of the switch-on periods to apply depends on the type of the ultrasonic probe 1d. It is necessary to determine the number of switching elements to switch on and/or to determine which switching elements to switch on since the number and relative positions of the lands 21 (23) are different among the different types of the ultrasonic probe 1d. The above-identified two tables may be combined to be used as one table. The main body 130 may alternatively include only one of the above-identified two tables. In this case, inputs may be combined as an input operation in a manner similar to the first embodiment. Still further, the ultrasonic probe identification table memory may be provided in the controller as a part of the features of the controller. In this case, the controller refers to the ultrasonic probe identification table memory for the controls.

The informing unit 130 gives a notice to an operator when the accumulated time has reached the predetermined time. As described above, the informing unit 130 is operative in response to the instruction signals from the accumulator 132 or the controls by the controller. The notice may be a suggestion that the ultrasonic probe 1d (i.e., the transducers 2) should be repolarized. The informing unit 130 may alternatively give a notice as a warning in a predetermined time before the time when the ultrasonic probe 1d should be repolarized. In this case, the notice may indicate when the ultrasonic probe 1d should be repolarized. Such an indication may be displayed as a message in a display provided for displaying ultrasound images, a typical component of the ultrasound imaging apparatus. The ultrasound imaging apparatus may alternatively warn it with a sound, such as, for example, beep sound or a voice message. Instead of giving notice as to when to repolarize, a remaining time until when the ultrasonic probe 1d should be repolarized may be calculated and estimated based on the accumulated time and be displayed in the display or be given as a sound warning.

When the operator inputs an instruction through the input unit in response to the notice given by the informing unit 131, the controller controls the switch unit and the high voltage power source so that the repolarization is implemented as described before.

Although the ultrasound imaging apparatus has been described only in the seventh to the ninth embodiments, any idea a of the embodiments described for the repolarization system (i.e., the first to the sixth embodiments) can also be applied to the ultrasound imaging apparatus.

The embodiments of the present invention described above are examples described only for making it easier to understand the present invention, and are not described for the limitation of the present invention. It is therefore to be understood that within the scope of the appended claims, the invention may be pracciced otherwise than as specifically described herein.

## Claims

1. A repolarization system (50) comprising an ultrasonic probe (1) having:
a plurality of piezo-electric transducers (2);
a plurality of electrode (5, 7) pairs connected to each piezo-electric transducer, respectively;
and a connector (22,24);
the repolarization system comprising:
a voltage generator (54) configured to supply a predetermined high voltage each of the plurality of electrode pairs in order to repolarise the plurality of piezo-electric transducers;
a switch unit (53) configured to control a supply of the predetermined high voltage to each of the electrode pairs;
an interface (56) configured to provide an instruction; and
a controller (55) configured to control the voltage generator to generate the predetermined high voltage and to control the switch unit (53) to supply the high voltage to each of the electrode pairs in accordance with the instruction;
wherein the connector (22, 24) is configured to connect each of the electrode pairs to the switch unit (53) through a plurality of lands (21), each of the lands corresponding to each of the electrode pairs;
the probe comprises a cable (26) connected between the switch and the connector;
each of the plurality of lands is sufficiently insulated from all the others to prevent a dielectric breakdown between the lands while each of the plurality of piezo-electric transducers is repolarized; and
the system is capable of coupling to an ultrasound imaging apparatus through the connector so as to display received echo signals through the ultrasonic probe on the ultrasound imaging apparatus.

2. The system according to Claim 1, wherein the ultrasonic probe is detachable from the system.

3. The system according to Claim 1, wherein the system is incorporated in the ultrasound imaging apparatus.

4. The system according to Claim 1, wherein the lands are provided at predetermined intervals so as to keep sufficient insulation between one and the next of the lands.

5. The system according to Claim 1, wherein an insulator is provided between one and the next of the lands.

6. The system according to Claim 1, further comprising a memory configured to store a table defining a relationship between ultrasonic probe type identification information and supply voltage information; and
an input unit configured to input the instruction including the ultrasonic probe type identification information, whereby the controller determines the predetermined high voltage based on the supply voltage information obtained in connection with the ultrasonic probe type information.

7. The system according to Claim 1, further comprising a memory configured to store a table defining a relationship between ultrasonic probe type identification information and switching condition information; and
an input unit configured to input the instruction including the ultrasonic probe type identification information, whereby the controller controls the switch unit based on the switching condition information obtained from the relationship between the ultrasonic probe type identification information and the switching condition information.

8. The system according to Claim 1, further comprising a memory configured to store a table defining a relationship between supply voltage type identification information and supply voltage information; and
an input unit configured to input the instruction including the supply voltage type identification information, whereby the controller determines the predetermined voltage based on the supply voltage information obtained from the relationship between the supply voltage type identification information and the supply voltage information.

9. The system according to Claim 1, further comprising an input unit configured to input the instruction including a value of the predetermined voltage.

10. The system according to Claim 1, further comprising an input unit configured to input a time period for which the predetermined voltage is supplied to the electrode pairs.

11. The system according to Claim 1, further comprising a memory configured to store a table defining a relationship between ultrasonic probe type identification information and supply voltage information, wherein the ultrasonic probe is further configured to store the ultrasonic probe type identification information, wherein the controller determines the predetermined voltage based on the supply voltage information obtained from the relationship between the ultrasonic probe type identification information and the supply voltage information, the ultrasonic probe type identification information being provided from the ultrasonic probe as the instruction.

12. The system according to Claim 1, further comprising a memory configured to store a table defining a relationship between ultrasonic probe type identification information and switching condition information, wherein the ultrasonic probe is further configured to store the ultrasonic probe type identification information, and wherein the controller controls the switch unit based on the switching condition information obtained from the relationship between the ultrasonic probe type identification information and the switching condition information, the ultrasonic probe type identification information being provided from the ultrasonic probe as the instruction.

13. The system according to Claim 1, further comprising a memory configured to store a table defining a relationship between supply voltage type identification information and supply voltage information, wherein the ultrasonic probe is further configured to store supply voltage type identification information, and wherein the controller determines the predetermined voltage based on the supply voltage information obtained from the relationship between the supply voltage type identification information and the supply voltage information, the supply voltage type identification information being provided from the ultrasonic probe as the instruction.

14. The system according to Claim 1, wherein the switch unit includes a plurality of switching elements, wherein the controller controls the switching elements to connect the voltage generator to the electrode pairs provided for each of the transducers, the each of the transducers corresponding to one of the switching elements.

15. The system according to Claim 1, wherein the controller is further configured to adjust a switch-on period of the switch unit in accordance with the instruction.

16. An ultrasound imaging apparatus for obtaining an ultrasound image through an ultrasonic probe (1), the apparatus comprising:
a plurality of electrode pairs (5, 7) provided in the ultrasonic probe;
a plurality of piezo-electric transducers (2) provided in the ultrasonic probe and configured to generate ultrasound so as to obtain the ultrasound image, each of the transducers being connected to each of the electrode pairs, respectively;
a voltage generator (54) configured to generate a predetermined voltage high enough to repolarize the transducers;
a switch unit (53) configured to control a supply of the predetermined voltage to the electrode pairs;
a connector (22, 24) provided in the probe and configured to connect the electrode pairs to the switch unit (53) through a plurality of lands, each of the lands corresponding to a different one of the electrode pairs, the lands being provided sufficiently insulated from each other to prevent a dielectric breakdown between the lands while repolarizing the transducers;
the probe comprising a cable (26) connected between the switch unit (53) and the connector;
the apparatus further comprising an interface (56) configured to provide an instruction; and
a controller (55) configured to control the voltage generator to generate the predetermined voltage and to control the switch unit (53) to supply the predetermined voltage to the electrodes in accordance with the instruction.

17. The apparatus according to Claim 16, further comprising an accumulator configured to provide accumulated time by accumulating time when the transducers generate the ultrasound; a recorder configured to record the accumulated time; and a notice component configured to provide a notice according to the accumulated time.

18. The apparatus according to Claim 17, wherein the notice component provides a notice when the accumulated time is equal to or greater than a predetermined time.

19. The apparatus according to Claim 18, wherein the notice component provides a notice for suggesting to repolarise the transducers.

20. The apparatus according to Claim 17, wherein the notice component provides the notice to suggest when the transducers are repolarized.

21. The apparatus according to Claim 17, wherein the notice component includes a display configured to display estimated remaining repolarizing time for the transducers based on the accumulated time.

22. The apparatus according to Claim 16, further comprising an accumulator configured to provide accumulated time by accumulating time when the transducers generate the ultrasound; and a recorder configured to record the accumulated time, wherein the controller is further configured to detect a power supply to the apparatus, and the controller automatically controls the switch unit to supply the predetermined voltage to the electrodes in response to detection of the power supply when the accumulated time is equal to or greater than a predetermined time.

23. The apparatus according to Claim 16, further comprising an accumulator configured to provide accumulated time by accumulating time when the transducers generate the ultrasound; and a recorder configured to record the accumulated time, wherein the controller is further configured to determine that the transducers terminate the ultrasound; and wherein the controller automatically controls the switch unit to supply the predetermined voltage to the electrodes in response to determination of a termination of the ultrasound when the accumulated time is equal to or greater than a predetermined time.

24. The apparatus according to Claim 17, wherein the accumulator and the recorder are provided in the ultrasonic probe.

25. The apparatus according to Claim 16, wherein the ultrasonic probe is detachable from the apparatus.

## Patentansprüche

1. Repolarisationssystem (50), eine Ultraschallsonde (1) umfassend, die Folgendes aufweist:
eine Vielzahl von piezoelektrischen Wandlern (2);
eine Vielzahl von Elektrodenpaaren (5, 7), die jeweilig an jeden piezoelektrischen Wandler angeschlossen sind;
und einen Verbinder (22, 24);
wobei das Repolarisationssystem umfasst:
einen Spannungsgenerator (54), dazu konfiguriert, jedes der Vielzahl von Elektrodenpaaren mit einer vorbestimmten Hochspannung zu versorgen, um die Vielzahl von piezoelektrischen Wandlern zu repolarisieren;
eine Schalteinheit (53), dazu konfiguriert, eine Versorgung von jedem der Elektrodenpaare mit der vorbestimmten Hochspannung zu steuern;
eine Schnittstelle (56), dazu konfiguriert, einen Befehl bereitzustellen; und
einen Controller (55), dazu konfiguriert, den Spannungsgenerator zu steuern, um eine vorbestimmte Hochspannung zu erzeugen und die Schalteinheit (53) zu steuern, um jedes der Elektrodenpaare gemäß dem Befehl mit der Hochspannung zu versorgen;
worin der Verbinder (22, 24) dazu konfiguriert ist, jedes der Elektrodenpaare durch eine Vielzahl von Anschlussflächen (21) an die Schalteinheit (53) anzuschließen, wobei jede der Anschlussflächen einem jeden der Elektrodenpaare entspricht;
die Sonde umfasst eine Kabel (26), das zwischen dem Schalter und dem Verbinder angeschlossen ist;
jedes der Vielzahl von Anschlussflächen ist von allen anderen ausreichend isoliert, um einen dielektrischen Durchschlag zwischen den Anschlussflächen zu verhindern, während jedes der Vielzahl von piezoelektrischen Wandlern repolarisiert wird; und
das System ist dazu befähigt, durch den Verbinder an eine Ultraschall-Abbildungsvorrichtung gekoppelt zu werden, um empfangene Echosignale durch die Ultraschallsonde auf der Ultraschall-Abbildungsvorrichtung darzustellen.

2. System nach Anspruch 1, worin die Ultraschallsonde vom System abnehmbar ist.

3. System nach Anspruch 1, worin das System in die Ultraschall-Abbildungsvorrichtung eingebaut ist.

4. System nach Anspruch 1, worin die Anschlussflächen mit vorbestimmten Abständen bereitgestellt werden, um ausreichende Isolation zwischen einer und der nächsten der Anschlussflächen aufrechtzuerhalten.

5. System nach Anspruch 1, worin zwischen einer und der nächsten der Anschlussflächen ein Isolator bereitgestellt wird.

6. System nach Anspruch 1, außerdem einen Speicher umfassend, der dazu konfiguriert ist, eine Tabelle zu speichern, die eine Beziehung zwischen Information zur Ultraschallsonden-Typidentifikation und Information zur Versorgungsspannung definiert; und
eine Eingangseinheit, dazu konfiguriert, den Befehl einzugeben, der die Information zur Ultraschallsonden-Typidentifikation enthält, wodurch der Controller die vorbestimmte Hochspannung auf der Basis der Information zur Versorgungsspannung bestimmt, die in Verbindung mit der Information zur Ultraschallsonden-Typidentifikation erlangt wird.

7. System nach Anspruch 1, außerdem einen Speicher umfassend, der dazu konfiguriert ist, eine Tabelle zu speichern, die eine Beziehung zwischen Information zur Ultraschallsonden-Typidentifikation und Information zum Schaltzustand definiert; und
eine Eingangseinheit, dazu konfiguriert, den Befehl einzugeben, der die Information zur Ultraschallsonden-Typidentifikation enthält, wodurch der Controller die Schalteinheit auf der Basis der Information zum Schaltzustand steuert, die aus der Beziehung zwischen der Information zur Ultraschallsonden-Typidentifikation und der Information zum Schaltzustand erlangt wird.

8. System nach Anspruch 1, außerdem einen Speicher umfassend, der dazu konfiguriert ist, eine Tabelle zu speichern, die eine Beziehung zwischen Information zur Versorgungsspannungs-Typidentifikation und Information zur Versorgungsspannung definiert; und
eine Eingangseinheit, dazu konfiguriert, den Befehl einzugeben, der die Information zur Versorgungsspannungs-Typidentifikation enthält, wodurch der Controller die vorbestimmte Spannung auf der Basis der Information zur Versorgungsspannung bestimmt, die aus der Beziehung zwischen der Information zur Versorgungsspannungs-Typidentifikation und der Information zur Versorgungsspannung erlangt wird.

9. System nach Anspruch 1, außerdem eine Eingangseinheit umfassend, die dazu konfiguriert ist, den Befehl einzugeben, der einen Wert der vorbestimmten Spannung enthält.

10. System nach Anspruch 1, außerdem eine Eingangseinheit umfassend, die dazu konfiguriert ist, eine Zeitperiode einzugeben, für die die Elektrodenpaare mit der vorbestimmten Spannung versorgt werden.

11. System nach Anspruch 1, außerdem einen Speicher umfassend, der dazu konfiguriert ist, eine Tabelle zu speichern, die eine Beziehung zwischen Information zur Ultraschallsonden-Typidentifikation und Information zur Versorgungsspannung definiert, worin die Ultraschallsonde außerdem dazu konfiguriert ist, die Information zur Ultraschallsonden-Typidentifikation zu speichern, worin der Controller die vorbestimmte Spannung auf der Basis der Information zur Versorgungsspannung bestimmt, die aus der Beziehung zwischen der Information zur Ultraschallsonden-Typidentifikation und der Information zur Versorgungsspannung erlangt wird, wobei die Information zur Ultraschallsonden-Typidentifikation von der Ultraschallsonde als der Befehl bereitgestellt wird.

12. System nach Anspruch 1, außerdem einen Speicher umfassend, der dazu konfiguriert ist, eine Tabelle zu speichern, die eine Beziehung zwischen Information zur Ultraschallsonden-Typidentifikation und Information zum Schaltzustand definiert, worin die Ultraschallsonde außerdem dazu konfiguriert ist, die Information zur Ultraschallsonden-Typidentifikation zu speichern und worin der Controller die Schalteinheit auf der Basis der Information zum Schaltzustand steuert, die aus der Beziehung zwischen der Information zur Ultraschallsonden-Typidentifikation und der Information zum Schaltzustand erlangt wird,
wobei die Information zur Ultraschallsonden-Typidentifikation von der Ultraschallsonde als der Befehl bereitgestellt wird.

13. System nach Anspruch 1, außerdem einen Speicher umfassend, der dazu konfiguriert ist, eine Tabelle zu speichern, die eine Beziehung zwischen Information zur Versorgungsspannungs-Typidentifikation und Information zur Versorgungsspannung definiert, worin die Ultraschallsonde außerdem dazu konfiguriert ist, Information zur Versorgungsspannungs-Typidentifikation zu speichern und
worin der Controller die vorbestimmte Spannung auf der Basis der Information zur Versorgungsspannung bestimmt, die aus der Beziehung zwischen der Information zur Versorgungsspannungs-Typidentifikation und der Information zur Versorgungsspannung erlangt wird, wobei die Information zur Versorgungsspannungs-Typidentifikation von der Ultraschallsonde als der Befehl bereitgestellt wird.

14. System nach Anspruch 1, worin die Schalteinheit eine Vielzahl von Schaltelementen enthält, worin der Controller die Schaltelemente steuert, um den Spannungsgenerator an die für jeden der Wandler bereitgestellten Elektrodenpaare anzuschließen, wobei jeder der Wandler einem der Schaltelemente entspricht.

15. System nach Anspruch 1, worin der Controller außerdem dazu konfiguriert ist, eine Einschaltperiode der Schalteinheit gemäß dem Befehl einzustellen.

16. Ultraschall-Abbildungsvorrichtung zum Erlangen eines Ultraschallbilds durch eine Ultraschallsonde (1), wobei die Vorrichtung umfasst:
eine Vielzahl von in der Ultraschallsonde bereitgestellten Elektrodenpaaren (5, 7);
eine Vielzahl von in der Ultraschallsonde bereitgestellten piezoelektrischen Wandlern (2), dazu konfiguriert, Ultraschall zu erzeugen, um das Ultraschallbild zu erhalten, wobei jeder der Wandler jeweilig an jedes der Elektrodenpaare angeschlossen ist;
einen Spannungsgenerator (54), dazu konfiguriert, eine vorbestimmte Spannung zu erzeugen, die hoch genug ist, um die Wandler zu repolarisieren;
eine Schalteinheit (53), dazu konfiguriert, eine Versorgung der Elektrodenpaare mit der vorbestimmten Spannung zu steuern;
einen in der Sonde bereitgestellten Verbinder (22, 24), dazu konfiguriert, die Elektrodenpaare durch eine Vielzahl von Anschlussflächen an die Schalteinheit (53) anzuschließen, wobei jede der Anschlussflächen einem verschiedenen der Elektrodenpaare entspricht, wobei die Anschlussflächen ausreichend voneinander isoliert bereitgestellt werden, um einen dielektrischen Durchschlag zwischen den Anschlussflächen während dem Repolarisieren der Wandler zu verhindern;
die Sonde, ein Kabel (26) umfassend, das zwischen der Schaltereinheit (53) und dem Verbinder angeschlossen ist;
die Vorrichtung, außerdem eine Schnittstelle (56) umfassend, die zum Bereitstellen eines Befehls konfiguriert ist; und
einen Controller (55), dazu konfiguriert, den Spannungsgenerator zu steuern, um die vorbestimmte Spannung zu erzeugen und die Schaltereinheit (53) zu steuern, um die die Elektroden gemäß dem Befehl mit der vorbestimmten Spannung zu versorgen.

17. Vorrichtung nach Anspruch 16, außerdem einen Akkumulator umfassend, der dazu konfiguriert ist, akkumulierte Zeit durch Akkumulieren der Zeit bereitzustellen, wenn die Wandler den Ultraschall erzeugen; ein Registriergerät, dazu konfiguriert, die akkumulierte Zeit zu registrieren; und eine Meldungskomponente, dazu konfiguriert, eine Meldung gemäß der akkumulierten Zeit bereitzustellen.

18. Vorrichtung nach Anspruch 17, worin die Meldungskomponente eine Meldung bereitstellt, wenn die akkumulierte Zeit größer oder gleich einer vorbestimmten Zeit ist.

19. Vorrichtung nach Anspruch 18, worin die Meldungskomponente eine Meldung bereitstellt, um zu empfehlen, dass die Wandler repolarisiert werden.

20. Vorrichtung nach Anspruch 17, worin die Meldungskomponente die Meldung bereitstellt, um zu empfehlen, wann die Wandler repolarisiert werden.

21. Vorrichtung nach Anspruch 17, worin die Meldungskomponente eine Anzeigeeinheit enthält, die zum Anzeigen der geschätzten verbleibenden Repolarisierungszeit für die Wandler auf der Basis der akkumulierten Zeit konfiguriert ist.

22. Vorrichtung nach Anspruch 16, außerdem einen Akkumulator umfassend, der zum Bereitstellen akkumulierter Zeit durch Akkumulieren der Zeit konfiguriert ist, wenn die Wandler den Ultraschall erzeugen; und ein Registriergerät, konfiguriert zum Registrieren der akkumulierten Zeit, worin der Controller außerdem dazu konfiguriert ist, eine Energieversorgung an die Vorrichtung zu detektieren und der Controller die Schalteinheit automatisch steuert, um die Elektroden mit der vorbestimmten Spannung zu versorgen, als Antwort auf das Detektieren der Energieversorgung, wenn die akkumulierte Zeit größer oder gleich einer vorbestimmten Zeit ist.

23. Vorrichtung nach Anspruch 16, außerdem einen Akkumulator umfassend, der zum Bereitstellen von akkumulierter Zeit durch Akkumulieren der Zeit konfiguriert ist, wenn die Wandler den Ultraschall erzeugen; und ein Registriergerät, konfiguriert zum Registrieren der akkumulierten Zeit, worin der Controller außerdem dazu konfiguriert ist, zu bestimmen, dass die Wandler den Ultraschall beenden; und worin der Controller die Schalteinheit automatisch steuert, um die Elektroden mit der vorbestimmten Spannung zu versorgen, als Antwort auf das Bestimmen einer Beendigung des Ultraschalls, wenn die akkumulierte Zeit größer oder gleich einer vorbestimmten Zeit ist.

24. Vorrichtung nach Anspruch 17, worin der Akkumulator und das Registriergerät in der Ultraschallsonde bereitgestellt werden.

25. Vorrichtung nach Anspruch 16, worin die Ultraschallsonde von der Vorrichtung abnehmbar ist.

## Revendications

1. Système de repolarisation (50) comportant une sonde ultrasonique (1) présentant;
une pluralité de transducteurs piézoélectriques (2);
une pluralité de paires d'électrodes (5, 7) connectées à chaque transducteur piézoélectrique, respectivement;
et un connecteur (22, 24);
le système de repolarisation comportant :
un générateur de tension (54) configuré pour fournir une tension élevée prédéterminée à chacune de la pluralité de pairs d'électrodes afin de repolariser la pluralité de transducteurs piézoélectriques;
une unité de commutateur (53) configurée pour commander un apport de la tension élevée prédéterminée à chacune des pairs d'électrodes;
une interface (56) configurée pour fournir une instruction; et
un contrôleur (55) configuré pour commander au générateur de tension de générer la tension élevée prédéterminée et pour commander à l'unité de commutateur (53) de fournir la tension élevée à chacune des pairs d'électrodes selon l'instruction;
dans lequel le connecteur (22, 24) est configuré pour connecter chacune des pairs d'électrodes à l'unité de commutateur (53) via une pluralité de pastilles d'interconnexion (21), chacune des pastilles d'interconnexion correspondant à chacune des pairs d'électrodes;
la sonde comporte un câble (26) connecté entre le commutateur et le connecteur;
chacune de la pluralité de pastilles d'interconnexion est suffisamment isolée de toutes les autres pour empêcher une rupture diélectrique entre les pastilles d'interconnexion lorsque chacune de la pluralité de transducteurs piézoélectriques est repolarisée; et
le système est apte à se coupler à un dispositif d'imagerie ultrasonore via le connecteur, de manière à afficher des signaux d'écho reçus via la sonde ultrasonique sur le dispositif d'imagerie ultrasonore.

2. Système selon la revendication 1, dans lequel la sonde ultrasonique est détachable du système.

3. Système selon la revendication 1, dans lequel le système est intégré dans le dispositif d'imagerie ultrasonore.

4. Système selon la revendication 1, dans lequel les pastilles d'interconnexion sont fournies à des intervalles prédéterminés afin de conserver suffisamment d'isolation entre une pastille d'interconnexion quelconque et la pastille suivante.

5. Système selon la revendication 1, dans lequel un isolant est délivré entre une pastille d'interconnexion quelconque et la pastille suivante.

6. Système selon la revendication 1, comportant en outre une mémoire configurée pour stocker une table définissant une relation entre une information d'identification de type de sonde ultrasonique et une information de tension d'alimentation; et
une unité d'entrée configurée pour entrer l'instruction comportant l'information d'identification de type de sonde ultrasonique, moyennant quoi le contrôleur détermine la tension élevée prédéterminée sur la base de l'information de tension d'alimentation obtenue conjointement avec l'information de type de sonde ultrasonique.

7. Système selon la revendication 1, comportant en outre une mémoire configurée pour stocker une table définissant une relation entre une information d'identification de type de sonde ultrasonique et une information de condition de commutation; et
une unité d'entrée configurée pour entrer l'instruction comportant l'information d'identification de type de sonde ultrasonique, moyennant quoi le contrôleur contrôle l'unité de commutateur sur la base de l'information de condition de commutation obtenue à partir de la relation entre l'information d'identification de type de sonde ultrasonique et l'information de condition de commutation.

8. Système selon la revendication 1, comportant en outre une mémoire configurée pour stocker une table définissant une relation entre une information d'identification de type de tension d'alimentation et une information de tension d'alimentation; et
une unité d'entrée configurée pour entrer l'instruction comportant l'information d'identification de type de tension d'alimentation, moyennant quoi le contrôleur détermine la tension prédéterminée sur la base de l'information de tension d'alimentation obtenue à partir de la relation entre l'information d'identification de type de tension d'alimentation et l'information de tension d'alimentation.

9. Système selon la revendication 1, comportant en outre une unité d'entrée configurée pour entrer l'instruction comportant une valeur de la tension prédéterminée.

10. Système selon la revendication 1, comportant en outre une unité d'entrée configurée pour entrer une période de temps pendant laquelle la tension prédéterminée est délivrée aux pairs d'électrodes.

11. Système selon la revendication 1, comportant en outre une mémoire configurée pour stocker une table définissant une relation entre une information d'identification de type de sonde ultrasonique et une information de tension d'alimentation, dans lequel la sonde ultrasonique est en outre configurée pour stocker l'information d'identification de type de sonde ultrasonique, dans lequel le contrôleur détermine la tension prédéterminée sur la base de l'information de tension d'alimentation obtenue à partir de la relation entre l'information d'identification de type de sonde ultrasonique et l'information de tension d'alimentation, l'information d'identification de type de sonde ultrasonique étant délivrée par la sonde ultrasonique en tant que l'instruction.

12. Système selon la revendication 1, comportant en outre une mémoire configurée pour stocker une table définissant une relation entre une information d'identification de type de sonde ultrasonique et une information de condition de commutation, dans lequel la sonde ultrasonique est en outre configurée pour stocker l'information d'identification de type de sonde ultrasonique, et dans lequel le contrôleur contrôle l'unité de commutateur sur la base de l'information de condition de commutation obtenue à partir de la relation entre l'information d'identification de type de sonde ultrasonique et l'information de condition de commutation, l'information d'identification de type de sonde ultrasonique étant délivrée par la sonde ultrasonique en tant que l'instruction.

13. Système selon la revendication 1, comportant en outre une mémoire configurée pour stocker une table définissant une relation entre une information d'identification de type de tension d'alimentation et une information de tension d'alimentation, dans lequel la sonde ultrasonique est en outre configurée pour stocker une information d'identification de type de tension d'alimentation, et dans lequel le contrôleur détermine la tension prédéterminée sur la base de l'information de tension d'alimentation obtenue à partir de la relation entre l'information d'identification de type de tension d'alimentation et l'information de tension d'alimentation, l'information d'identification de type de tension d'alimentation étant délivrée par la sonde ultrasonique en tant que l'instruction.

14. Système selon la revendication 1, dans lequel l'unité de commutateur comporte une pluralité d'éléments de commutation, dans lequel le contrôleur contrôle les éléments de commutation pour connecter le générateur de tension aux pairs d'électrodes délivrées pour chacun des transducteurs, lesdits chacun des transducteurs correspondant à l'un des éléments de commutation.

15. Système selon la revendication 1, dans lequel le contrôleur est en outre configuré pour régler une période de commutation active de l'unité de commutateur selon l'instruction.

16. Dispositif d'imagerie ultrasonore pour obtenir une image ultrasonore via une sonde ultrasonique (1), le dispositif comportant :
une pluralité de pairs d'électrodes (5, 7) délivrées dans la sonde ultrasonique;
une pluralité de transducteurs piézoélectriques (2) délivrés dans la sonde ultrasonique et configurés pour générer des ultrasons afin d'obtenir l'image ultrasonore, chacun des transducteurs étant connecté à chacune des pairs d'électrodes, respectivement;
un générateur de tension (54) configuré pour générer une tension prédéterminée suffisamment élevée pour repolariser les transducteurs;
une unité de commutateur (53) configurée pour commander un apport de la tension prédéterminée aux pairs d'électrodes;
un connecteur (22, 24) délivré dans la sonde et configuré pour connecter les pairs d'électrodes à l'unité de commutateur (53) via une pluralité de pastilles d'interconnexion, chacune des pastilles d'interconnexion correspondant à une paire distincte des pairs d'électrodes, les pastilles d'interconnexion étant délivrées suffisamment isolées les unes des autres pour éviter une rupture diélectrique entre les pastilles d'interconnexion lors de la repolarisation des transducteurs;
la sonde comportant un câble (26) connecté entre l'unité de commutateur (53) et le connecteur;
le dispositif comportant en outre une interface (56) configurée pour délivrer une instruction; et
un contrôleur (55) configuré pour commander au générateur de tension de générer la tension prédéterminée et pour commander à l'unité de commutateur (53) de délivrer la tension prédéterminée aux électrodes selon l'instruction.

17. Dispositif selon la revendication 16, comportant en outre un accumulateur configuré pour délivrer un temps accumulé en accumulant un temps où les transducteurs génèrent les ultrasons; un enregistreur configuré pour enregistrer le temps accumulé, et un composant de notification configuré pour délivrer une notification selon le temps accumulé.

18. Dispositif selon la revendication 17, dans lequel le composant de notification délivre une notification lorsque le temps accumulé est égal ou supérieur à un temps prédéterminé.

19. Dispositif selon la revendication 18, dans lequel le composant de notification délivre une notification pour suggérer de repolariser les transducteurs.

20. Dispositif selon la revendication 17, dans lequel le composant de notification délivre une notification pour suggérer à quel moment les transducteurs doivent être repolarisés.

21. Dispositif selon la revendication 17, dans lequel le composant de notification inclut un écran d'affichage configuré pour afficher le temps de repolarisation restant estimé pour les transducteurs sur la base du temps accumulé.

22. Dispositif selon la revendication 16, comprenant en outre un accumulateur configuré pour délivrer le temps accumulé en accumulant le temps où les transducteurs génèrent l'ultrason; et un enregistreur configuré pour enregistrer le temps accumulé, le contrôleur étant en outre configuré pour détecter une alimentation électrique vers le dispositif et contrôler automatiquement l'unité de commutateur pour fournir la tension prédéterminée aux électrodes en réponse à la détection de l'alimentation électrique quand le temps accumulé est égal ou supérieur à un temps prédéterminé.

23. Dispositif selon la revendication 16, comprenant en outre un accumulateur configuré pour délivrer le temps accumulé en accumulant un temps où les transducteurs génèrent les ultrasons; et un enregistreur configuré pour enregistrer le temps accumulé, le contrôleur étant en outre configuré pour déterminer que les transducteurs terminent l'ultrason; et le contrôleur contrôlant automatiquement l'unité de commutateur pour fournir la tension prédéterminée aux électrodes en réponse à une constatation d'un arrêt des ultrasons quand le temps accumulé est égal ou supérieur à un temps prédéterminé.

24. Dispositif selon la revendication 17, dans lequel l'accumulateur et l'enregistreur sont fournis dans la sonde ultrasonique.

25. Dispositif selon la revendication 16, dans lequel la sonde ultrasonique est détachable du dispositif.
